# EUROPEAN PATENT APPLICATION

(11) **EP 4 617 379 A1**
(43) Date of publication of application: **17.09.2025**
(21) Application number: 25163410.1
(22) Date of filing: 13.03.2025
(51) Int. Cl.: C12Q 1/6806, C12Q 1/6848, C12Q 1/70

(54) **COMPOSITION FOR PRETREATMENT AND METHOD FOR PRETREATMENT OF TARGET SAMPLE OF NUCLEIC ACID DETECTION**

(30) Priority: 15.03.2024 JP 2024041420
(71) Applicant: Yokogawa Electric Corporation, Tokyo 180-8750 (JP)
(72) Inventor: Aoki, Hidetoshi, Musashino-shi, Tokyo 180-8750 (JP); Miyauchi, Yuki, Musashino-shi, Tokyo 180-8750 (JP); Maemura, Chika, Musashino-shi, Tokyo 180-8750 (JP)
(74) Representative: Grünecker Patent- und Rechtsanwälte PartG mbB

(57) **Abstract**

The present disclosure concerns detection of a target nucleic acid comprised in a sample, in particular, a water sample in the environment, such as treated sewage water or rivers, in a more convenient manner with higher accuracy. A target sample of nucleic acid detection is mixed with a composition for pretreatment comprising EDTA and the resultant is then heated using a heating means maintained at 90°C or higher.

## Description

### CROSS REFERENCE TO RELATED APPLICATIONS

The present application claims priority from Japanese patent application JP 2024-041420 filed on March 15, 2024, the content of which is hereby incorporated by reference into this application.

### BACKGROUND

### Technical Field

The present disclosure relates to a composition for pretreatment and a method for pretreatment used to detect a target nucleic acid in a sample comprising nucleic acids.

### Background Art

As a contamination indicator of the water environment such as rivers, the quantified viral load in water has been used. Concerning the water environment, in recent years, a viral load in sewage water has been reported to serve as a prognostic indicator of an infectious disease epidemic, and such indicator has been used by public administration to develop countermeasures against infectious diseases. Quantitative measurement of environmental viruses to identify the contamination status of rivers or tap and sewage water has been primarily performed by quantitative PCR. Pretreatment of samples used therefor necessitates extraction and purification of viral nucleic acids. In the case of RNA viruses, in addition, it is necessary to perform a step of RNA reverse transcription.

In the step of pretreatment of nucleic acids, it is necessary that complicated reaction procedures be performed in combination. For simplification of procedures, commercially available kits for nucleic acid extraction are often used to extract viral nucleic acids from samples; however, it would generally take approximately 1 hour to perform nucleic acid extraction. When measuring samples in the environment, it is necessary that samples be collected from many measurement points and measurement be continuously performed. Accordingly, it is necessary that pretreatment of samples be performed in a simple manner, within a short period of time, and at a low cost, to the maximum extent possible.

JP 2008-72904 A discloses a method for amplifying and detecting nucleic acids of microorganisms in a sample comprising a large quantity of contaminants, such as contaminated food materials, with the addition of metal ions to the sample while avoiding the influence of food ingredients or the like. Also, JP 2012-157265 A discloses a method of heat-treating a sample in the water environment to extract viral nucleic acids in a convenient and rapid manner. Further, M. Watanabe et al., Plant Biotechnology 33, 133-136, 2016 discloses that the efficiency for nucleic acid amplification is improved with the addition of polyvinyl pyrrolidone (PVPP) to a sample comprising a large quantity of contaminants.

### SUMMARY

The present disclosure relates to detection of a target nucleic acid comprised in a sample, in particular, a water sample in the environment, such as a sample in treated sewage water or rivers, in a more convenient and accurate manner. More particularly, the present disclosure relates to detection of a nucleic acid under conditions that are less susceptible to the influence of a nuclease comprised in the sample.

The present disclosure provides the following.
[1] A method for pretreatment of a target sample of nucleic acid detection comprising:
   i) a step of mixing a sample with a composition for pretreatment comprising ethylenediaminetetraacetic acid (EDTA); and
   ii) a step of heating the sample mixed in step i) using a heating means maintained at 90°C or higher.
[2] The method according to [1], wherein the sample is a water sample in the environment.
[3] The method according to [1] or [2], wherein the nucleic acid is a viral nucleic acid.
[4] The method according to any of [1] to [3], which comprises a step of concentrating nucleic acids in a sample before step i).
[5] The method according to any of [1] to [4], wherein the concentration of EDTA in the sample mixed in step i) is 0.01 to 20 mM.
[6] The method according to [5], wherein the concentration of EDTA in the sample mixed in step i) is 0.1 to 10 mM.
[7] The method according to any of [1] to [6], wherein a duration time of heat treatment in step ii) is less than 120 seconds.
[8] The method according to any of [1] to [7], wherein temperature of the heating means used in step ii) is 105°C to 160°C.
[9] A method for detecting a nucleic acid in a sample comprising:
   a) a step of mixing a target sample of nucleic acid detection with a composition for pretreatment comprising EDTA;
   b) a step of heating the sample after step a) using a heating means maintained at 90°C or higher;
   c) a step of mixing the sample after step b) with a composition for nucleic acid detection; and
   d) a step of detecting a nucleic acid in the sample after step c).
[10] The method according to [9], wherein the composition for nucleic acid detection is a composition for a reverse transcription polymerase chain reaction (RT-PCR).
[11] The method according to [9] or [10], wherein a duration time from after step b) to before step d) is less than 4 hours.
[12] The method according to any of [9] to [11], wherein the concentration of EDTA in the sample mixed in step c) is 0.001 to 10 mM.
[13] A composition comprising EDTA, which is used for pretreatment of a target sample of nucleic acid detection.
[14] The composition according to [13], wherein the concentration of EDTA is 0.01 to 20 mM.
[15] The composition according to [14], wherein the concentration of EDTA is 0.1 to 10 mM.
[16] The composition according to any of [13] to [15], wherein the sample is a water sample in the environment.
[17] The composition according to any of [13] to [16], wherein the nucleic acid is a viral nucleic acid.
[18] The composition according to any of [13] to [17], which further comprises a buffer.

The composition and the method according to the present disclosure enable detection of a target nucleic acid comprised in a sample, in particular, a water sample in the environment, such as a sample in treated sewage water or rivers, in a more convenient and accurate manner.

### BRIEF DESCRIPTION OF THE DRAWINGS

Fig. 1 shows a chart demonstrating a correlation between the period of heat treatment of the sample and the quantified PMMoV load found in Comparative Example 1.
Fig. 2 shows a chart demonstrating a correlation between the static period from after heat treatment to before reverse transcription and the quantified PMMoV load found in Comparative Example 1.
Fig. 3 shows a chart demonstrating a correlation between the period of heat treatment of the sample and the quantified PMMoV load found in Comparative Example 2.
Fig. 4 shows a chart demonstrating a correlation between the static period from after heat treatment to before reverse transcription and the quantified PMMoV load with or without the addition of EDTA found in Example 1.
Fig. 5 shows a chart demonstrating a correlation between the conditions for heat treatment of Samples A to C (at 95°C for 5 minutes or at 140°C for 15 seconds) and the quantified PMMoV load found in Example 2.

### DETAILED DESCRIPTION

### 1. Definition

The term "pretreatment" used herein refers to treatment to make a nucleic acid in a target sample of nucleic acid detection to be suitable for amplification before a step of nucleic acid detection (a step of nucleic acid amplification, in particular). More particularly, the term refers to treatment to extract a nucleic acid from a viral particle or cell. The term "quantified load" of viruses used herein refers to a numerical value equivalent to a virus count relative to a given volume of a sample (e.g., 1 µl or 5 µl), which is determined based on the performance number of a reference solution comprising viral nucleic acids at known concentration. The term "composition" used herein encompasses both a compound consisting of a single component and a mixture comprising a plurality of components.

The term "ethylenediaminetetraacetic acid (EDTA)" used herein encompasses all of free acid, metal salt, and solvate forms. While EDTA may be in any of such forms, EDTA in the form of a water-soluble metal salt may generally be used. Specifically, EDTA disodium or EDTA dipotassium may be used. When preparing an aqueous solution of EDTA, a solvate of the metal salt, in particular, a hydrate of the metal salt can be used.

A target "sample" of nucleic acid detection is not particularly limited herein, provided that such sample can comprise nucleic acids. Examples thereof include samples in the environment, such as samples in rivers, treated sewage water, active sludge, and soil, samples involved in quality control of food, beverage, medical, and cosmetic products, biological samples collected from animals and plants, and samples of cultured microorganisms. In particular, samples in the environment that can comprise unknown components capable of inhibiting nucleic acid amplification may be used.

The term "nucleic acid" used herein encompasses all nucleic acids derived from viral, bacterial, fungal, and other eukaryotic cells. For example, a virus-derived nucleic acid may be genomic DNA or genomic RNA, and it may be in the form of any of single-stranded DNA, single-stranded RNA, double-stranded DNA, and double-stranded RNA. Nucleic acids derived from bacterial, fungal, and other eukaryotic cells may be in the form of any of, for example, genomic DNA, ribosomal RNA, messenger RNA, and plasmid DNA. Examples of viruses include plant-infecting viruses detected as environmental contamination indicators in sewage water, such as Pepper mild mottle virus (PMMoV), and human-infecting viruses, such as rotavirus, adenovirus, norovirus, RS virus, human metapneumovirus, and SARS-CoV-2. Examples of bacteria include bacteria of the genera *Acinetobacter* sp., *Actinomyces* sp., *Aerococcus* sp., *Aeromonas* sp., *Alcaligenes* sp., *Bacillus* sp., *Bacteroides* sp., *Bordetella* sp., *Branhamella* sp., *Brevibacterium* sp., *Campylobacter* sp., *Candida* sp., *Capnocytophaga* sp., *Chromobacterium* sp., *Clostridium* sp., *Corynebacterium* sp., *Cryptococcus* sp., *Deinococcus* sp., *Enterococcus* sp., *Erysielothrix* sp., *Escherichia* sp., *Flavobacterium* sp., *Gemella* sp., *Haemophilus* sp., *Klebsiella* sp., *Lactobacillus* sp., *Lactococcus* sp., *Legionella* sp., *Leuconostoc* sp., *Listeria* sp., *Micrococcus* sp., *Mycobacterium* sp., *Neisseria* sp., *Cryptosporidium* sp., *Nocardia* sp., *Oerskovia* sp., *Paracoccus* sp., *Pediococcus* sp., *Peptostreptococcus* sp., *Propionibacterium* sp., *Proteus* sp., *Pseudomonas* sp., *Rahnella* sp., *Rhodococcus* sp., *Rhodospirillium* sp., *Staphylococcus* sp., *Streptomyces* sp., *Streptococcus* sp., *Vibrio* sp., and *Yelsinia* sp.

A "heating means" is not particularly limited herein, provided that such means is capable of heating a container wall or the like to 90°C or higher. Examples of heating means include a block heater equipped with Peltier devices, an oil bath, hot air, and the like.

A technique of "nucleic acid detection" is not particularly limited herein, and it may be any of known techniques, such as nucleic acid amplification, Northern hybridization, Southern hybridization, and the DNA probe method. Preferably, a method of nucleic acid amplification that enables detection with high sensitivity may be employed. A technique of "nucleic acid amplification" is not particularly limited herein, provided that a particular nucleotide sequence of the template nucleic acid as a detection target can be amplified. For example, any of PCR (polymerase chain reaction), LAMP (loop-mediated isothermal amplification), and SDA (strand displacement amplification) may be employed. When a target nucleic acid of detection is RNA, preferably, reverse transcription may be performed before nucleic acid amplification.

### 2. Composition for pretreatment of target sample of nucleic acid detection

The first embodiment of the present disclosure relates to a composition used for pretreatment of a target sample of nucleic acid detection. The composition of the present embodiment comprises ethylenediaminetetraacetic acid (EDTA). The composition of the present embodiment can prevent a nucleic acid-comprising sample from being affected by a nuclease or the like comprised in a sample. This enables detection with high sensitivity and high accuracy in a following step of nucleic acid detection.

Preferably, the composition of the present embodiment may be used for pretreatment of a water sample in the environment. The composition of the present embodiment can avoid the influence of a nuclease or the like comprised in a sample. Accordingly, such composition may be used for detection of a nucleic acid in a sample in the environment, in particular, a water sample in the environment, such as a sample in rivers, treated sewage water, or the like, comprising unknown components. Since the composition of the present embodiment enables detection of a nucleic acid in a water sample in the environment with high sensitivity and high accuracy, it is possible to identify the conditions of infectiousness and contamination of the water environment. Preferably, the composition of the present embodiment may be used to detect a nucleic acid derived from a source of viral, bacterial, or other infection. Preferably, the composition of the present embodiment may be used to detect a nucleic acid derived from a virus.

The concentration of EDTA comprised in the composition of the present embodiment may be 0.01 to 20 mM. Preferably, such concentration may be 0.1 to 10 mM. The concentration of EDTA may be, for example, 0.2 mM or higher, 0.3 mM or higher, 0.4 mM or higher, 0.5 mM or higher, 0.6 mM or higher, 0.7 mM or higher, 0.8 mM or higher, 0.9 mM or higher, 1.0 mM or higher, 1.1 mM or higher, 1.2 mM or higher, 1.3 mM or higher, 1.4 mM or higher, 1.5 mM or higher, 1.6 mM or higher, 1.7 mM or higher, 1.8 mM or higher, 1.9 mM or higher, 2.0 mM or higher, 2.1 mM or higher, 2.2 mM or higher, 2.3 mM or higher, 2.4 mM or higher, 2.5 mM or higher, 3.0 mM or higher, 3.5 mM or higher, 4.0 mM or higher, 4.5 mM or higher, 5.0 mM or higher, 6.0 mM or higher, 7.0 mM or higher, 8.0 mM or higher, or 9.0 mM or higher. The concentration of EDTA may be, for example, 9.5 mM or lower, 9.0 mM or lower, 8.5 mM or lower, 8.0 mM or lower, 7.5 mM or lower, 7.0 mM or lower, 6.9 mM or lower, 6.8 mM or lower, 6.7 mM or lower, 6.6 mM or lower, 6.5 mM or lower, 6.4 mM or lower, 6.3 mM or lower, 6.2 mM or lower, 6.1 mM or lower, 6.0 mM or lower, 5.9 mM or lower, 5.8 mM or lower, 5.7 mM or lower, 5.6 mM or lower, 5.5 mM or lower, 5.4 mM or lower, 5.3 mM or lower, 5.2 mM or lower, 5.1 mM or lower, 5.0 mM or lower, 4.9 mM or lower, 4.8 mM or lower, 4.7 mM or lower, 4.6 mM or lower, 4.5 mM or lower, 4.4 mM or lower, 4.3 mM or lower, 4.2 mM or lower, 4.1 mM or lower, 4.0 mM or lower, 3.9 mM or lower, 3.8 mM or lower, 3.7 mM or lower, 3.6 mM or lower, 3.5 mM or lower, 3.4 mM or lower, 3.3 mM or lower, 3.2 mM or lower, 3.1 mM or lower, 3.0 mM or lower, 2.5 mM or lower, 2.0 mM or lower, 1.5 mM or lower, or 1.0 mM or lower.

In the reaction system used in a following step of nucleic acid detection and, in particular, in the reaction system used in nucleic acid amplification, the concentration of EDTA in the composition for pretreatment may be diluted to 1.5- to 20-fold from the concentration indicated above. Preferably, the concentration of EDTA may be diluted to 2.0- to 10-fold from the concentration indicated above.

Preferably, the composition of the present embodiment may comprise a buffer. The composition may comprise a pH buffer. A known buffer may be used herein because such buffer would not adversely affect the reaction system used in nucleic acid detection described below. Examples of buffers that can be used include pH buffers, such as HEPES, BES, TES, MOBS, DIPSO, TAPSO, HEPPSO, TAPSO, TEA, glycylglycine, and bicine, in addition to Tris-HCl (pH 7.0 to 8.5). Preferably, a pH level of the composition of the present embodiment may be 6.0 to 9.0. More preferably, a pH level may be 6.5 to 8.5.

The composition of the present embodiment may further comprise other ingredients. Examples of other ingredients include substances used to expose nucleic acids from capsids or cells. Specific examples thereof include alkaline substances (e.g., NaOH and KOH), acids (e.g., HCl and H₂SO₄), enzymes (e.g., proteolytic enzyme, such as Proteinase K, and polysaccharide-degrading enzymes, such as chitinase, lysozyme, and zymolyase), surfactants (e.g., anionic surfactant, such as SDS, cationic surfactant, such as CTAB (cetyltrimethylammonium bromide), nonionic surfactant, such as Triton-X, and zwitterionic surfactant, such as betaine (a generic term for a compound having a particular structure, such as trimethylglycine)), redox agents (e.g., hydrogen peroxide solution, β-mercaptoethanol, and dithiothreitol), and protein denaturants (e.g., guanidinium hydrochloride and urea). A plurality of ingredients indicated above may be used in combination. When a sample comprises a large quantity of contaminants other than nucleic acids, for example, polyvinyl pyrrolidone (PVPP) may be added to adjust the final concentration thereof to approximately 50 mg/ml at the time of nucleic acid detection (see M. Watanabe et al., Plant Biotechnology 33, 133-136, 2016).

### 3. Method for pretreatment of target sample of nucleic acid detection

The second embodiment of the present disclosure relates to a method for pretreatment of a target sample of nucleic acid detection. The method of the present embodiment comprises: i) a step of mixing a sample with a composition for pretreatment comprising EDTA; and ii) a step of heating the sample mixed in step i) using a heating means maintained at 90°C or higher.

In the method of the present embodiment, a sample may be a water sample in the environment, such as a sample in rivers, treated sewage water, or the like. In some embodiments, a nucleic acid as a detection target may be derived from a virus.

### 3-1. Step of preparation

Hereafter, each step of the method of the present embodiment is described. While the method of the present embodiment comprises steps i) and ii) described above as essential steps, the method may comprise at least one step of preparation before step i). When a sample comprises a large quantity of impurities in addition to nucleic acids, for example, the method may comprise a step of eliminating such impurities. An example of such step is filtration using a coarse mesh filter or filter paper. When a sample has a high solid content, such as some food products or biological samples, for example, the method may comprise a step of fragmenting a solid content in a solution and a step of eliminating insoluble ingredients (residues).

When the concentration of nucleic acids is low in a sample, such as a water sample in the environment, in particular, the method may comprise a step of concentrating nucleic acids in the sample. A technique for concentrating a sample, which is not particularly limited, include concentration by ultrafiltration, adsorption of nucleic acids onto a filter made of a glass fiber or a material similar thereto, and nucleic acid deposition with the aid of ethanol or isopropanol.

### 3-2. Step i) Step of mixing with EDTA

Step i) of the present embodiment comprises mixing a sample with a composition for pretreatment comprising EDTA. As a composition for pretreatment comprising EDTA, the composition described in "2. Composition for pretreatment of target sample of nucleic acid detection" section above may be used.

In step i), the concentration of EDTA in the sample after mixing may be 0.01 to 20 mM. Prederably, the concentration of EDTA may be 0.1 to 10 mM. More preferably, the concentration of EDTA may be 1 to 5 mM. Most preferably, the concentration of EDTA may be 2.5 mM. The concentration of EDTA may be, for example, 0.2 mM or higher, 0.3 mM or higher, 0.4 mM or higher, 0.5 mM or higher, 0.6 mM or higher, 0.7 mM or higher, 0.8 mM or higher, 0.9 mM or higher, 1.0 mM or higher, 1.1 mM or higher, 1.2 mM or higher, 1.3 mM or higher, 1.4 mM or higher, 1.5 mM or higher, 1.6 mM or higher, 1.7 mM or higher, 1.8 mM or higher, 1.9 mM or higher, 2.0 mM or higher, 2.1 mM or higher, 2.2 mM or higher, 2.3 mM or higher, 2.4 mM or higher, 2.5 mM or higher, 3.0 mM or higher, 3.5 mM or higher, 4.0 mM or higher, 4.5 mM or higher, 5.0 mM or higher, 6.0 mM or higher, 7.0 mM or higher, 8.0 mM or higher, or 9.0 mM or higher. The concentration of EDTA may be, for example, 9.5 mM or lower, 9.0 mM or lower, 8.5 mM or lower, 8.0 mM or lower, 7.5 mM or lower, 7.0 mM or lower, 6.9 mM or lower, 6.8 mM or lower, 6.7 mM or lower, 6.6 mM or lower, 6.5 mM or lower, 6.4 mM or lower, 6.3 mM or lower, 6.2 mM or lower, 6.1 mM or lower, 6.0 mM or lower, 5.9 mM or lower, 5.8 mM or lower, 5.7 mM or lower, 5.6 mM or lower, 5.5 mM or lower, 5.4 mM or lower, 5.3 mM or lower, 5.2 mM or lower, 5.1 mM or lower, 5.0 mM or lower, 4.9 mM or lower, 4.8 mM or lower, 4.7 mM or lower, 4.6 mM or lower, 4.5 mM or lower, 4.4 mM or lower, 4.3 mM or lower, 4.2 mM or lower, 4.1 mM or lower, 4.0 mM or lower, 3.9 mM or lower, 3.8 mM or lower, 3.7 mM or lower, 3.6 mM or lower, 3.5 mM or lower, 3.4 mM or lower, 3.3 mM or lower, 3.2 mM or lower, 3.1 mM or lower, 3.0 mM or lower, 2.5 mM or lower, 2.0 mM or lower, 1.5 mM or lower, or 1.0 mM or lower. By adjusting the concentration to the level indicated above, it is possible to sufficiently suppress degradation of a nucleic acid, in particular, RNA. By adequately diluting EDTA, in addition, it is possible to detect a target nucleic acid while preventing EDTA from inhibiting the reaction or without lowering sensitivity because of excessive dilution in the following step of detection.

Preferably, a composition for pretreatment comprising EDTA may comprise a buffer, in particular, a pH buffer, in addition to EDTA. A known buffer may be used herein because such buffer would not adversely affect the reaction system used in nucleic acid detection described below. Examples of buffers that can be used include pH buffers with a buffering capacity in a pH range of 6 to 9, such as Tris-HCl (pH 7.0 to 8.5), HEPES, BES, TES, MOBS, DIPSO, TAPSO, HEPPSO, TAPSO, TEA, glycylglycine, and bicine. Preferably, a pH level of the sample after mixing may be 6.0 to 9.0. More preferably, such pH level may be 6.5 to 8.5.

The composition for pretreatment may further comprise other ingredients. Examples of the other ingredients include substances used to expose nucleic acids from capsids or cells. Specific examples thereof include alkaline substances (e.g., NaOH and KOH), acids (e.g., HCl and H₂SO₄), enzymes (e.g., proteolytic enzyme, such as Proteinase K, and polysaccharide-degrading enzymes, such as chitinase, lysozyme, and zymolyase), surfactants (e.g., anionic surfactant, such as SDS, cationic surfactant, such as CTAB (cetyltrimethylammonium bromide), nonionic surfactant, such as Triton-X, and zwitterionic surfactant, such as betaine (a generic term for a compound having a particular structure, such as trimethylglycine)), redox agents (e.g., hydrogen peroxide solution, β-mercaptoethanol, and dithiothreitol), and protein denaturants (e.g., guanidinium hydrochloride and urea). A plurality of ingredients indicated above may be used in combination.

In step i), the concentration of nucleic acids in the sample after mixing is not particularly limited. Preferably, such concentration may be 1 fg/µl to 500 ng/µl. In some other embodiments, it may be 0.1 pg/µl to 100 ng/µl.

### 3-3. Step ii) Step of heating

Step ii) of the method of the present embodiment comprises heating the sample mixed in step i) using a heating means maintained at 90°C or higher. The method of the present embodiment comprises the step of heating. Accordingly, a sample can be prepared for nucleic acid detection within a short period of time.

In step ii), heating may be performed at 100°C or higher, in particular, 105°C to 160°C, or 125°C to 145°C. In such a case, a sample may be sealed in a container that is tolerant to sealed heating and it may then be heated in that state. Specifically, a heat-conductive, heat-tolerant, and pressure-tolerant container that can be sealed may be used. In step ii), a sample to be heated is an aqueous solution. When a container is not sealed, it is difficult to bring the temperature to higher than 100°C. By heating the sample in a sealed container, the temperature and the pressure are elevated inside the container. It is accordingly possible to heat the sample to the temperature to higher than 100°C. Examples of containers include a boil-lock type polypropylene tube (capacity: 0.2 ml, 0.6 ml, 1.5 ml, etc.) having a mechanical structure for sealing purposes, a heat-sealable bag, a glass test tube with a screw cap, and a microfluidic channel chip. When heating is performed at 90°C to 100°C, a common polypropylene tube or the like can be adequately used as a container.

In order to elevate the temperature of a sample with higher certainty, the inside of the container may be conditioned to be free from a gas phase. For example, the container can be filled with a sample solution, so that air bubbles or the gas phase would not remain therein, and the container can then be hermetically sealed. Alternatively, a high-boiling-point solvent, such as mineral oil, may be introduced into the gas phase in the container.

When heating is performed at 90°C to 100°C in step ii), a period of heat treatment needs to be approximately 5 minutes. If the temperature is elevated to 100°C or higher, however, a period of heating may be less than 120 seconds. Preferably, a period of heating may be less than 45 seconds, less than 35 seconds, or less than 25 seconds. More preferably, a period of heating may be 15 seconds or less. Heating may be performed by preheating the heating means to a designated temperature in advance and mounting the container on the heating means for a designated period of time only. After the completion of heating for a designated period of time, the sample may be cooled immediately.

The heated sample may be cooled to room temperature (e.g., 20°C to 30°C), refrigeration temperature (e.g., 2°C to 8°C), or ice cooling temperature. When it is difficult to perform nucleic acid amplification immediately thereafter, in particular, the sample may be stored at refrigeration temperature or lower immediately.

When a nucleic acid derived from a cell with a hard structure, such as a Gram-positive bacterium or fungus, is targeted, the step of heating may be performed two times or more. When a nucleic acid derived from a virus from the water environment is targeted, in contrast, the duration time of the step of heating may not be extended, so as to avoid the influence of a nuclease or the like in the sample. Accordingly, the step of heating may be performed only once.

### 4. Method for detecting nucleic acid in sample

The third embodiment of the present disclosure relates to a method for detecting a nucleic acid in a sample. The method of the present embodiment comprises:
a) a step of mixing a target sample of nucleic acid detection with a composition for pretreatment comprising EDTA;
b) a step of heating the sample after step a) using a heating means maintained at 90°C or higher;
c) a step of mixing the sample after step b) with a composition for nucleic acid detection; and
d) a step of detecting a nucleic acid in the sample after step c).

The method of the present embodiment can further comprise subjecting the sample pretreated in "3. Method for pretreatment of target sample of nucleic acid detection" to nucleic acid detection, unless it is otherwise described or unless there is inconsistency.

### 4-1. Step of preparation

Hereafter, each step of the method of the present embodiment is described. While the method of the present embodiment comprises steps a) to d) described above as essential steps, the method may comprise at least one step of preparation before step a). When a sample comprises a large quantity of impurities in addition to nucleic acids, for example, the method may comprise a step of eliminating such impurities. An example of such step is filtration using a coarse mesh filter or filter paper.

When the concentration of nucleic acids is low in a sample, such as a water sample in the environment, in particular, the method may comprise a step of concentrating nucleic acids in the sample.

### 4-2. Step a) Step of mixing with EDTA

Step a) of the present embodiment comprises mixing a sample with a composition for pretreatment comprising EDTA. As a composition for pretreatment comprising EDTA, the composition described in "2. Composition for pretreatment of target sample of nucleic acid detection" section above may be used. In step a), the concentration of EDTA in the sample after mixing may be 0.01 to 20 mM. Preferably, the concentration of EDTA may be 0.1 to 10 mM.

In some embodiments, a composition for pretreatment comprising EDTA may comprise a buffer, in particular, a pH buffer, in addition to EDTA. Any buffer may be used herein unless the buffer would adversely affect the reaction system used in nucleic acid detection described below. A buffer may be a pH buffer. Preferably, a pH level of the mixed sample may be 6.0 to 9.0. More preferably, such pH level may be 6.5 to 8.5. The composition for pretreatment may further comprise other ingredients. Examples of other ingredients include substances used to expose nucleic acids from capsids or cells.

### 4-3. Step b) Step of heating

Step b) of the present embodiment comprises heating the sample after step a) using a heating means maintained at 90°C or higher. In step b), a sample may be sealed in a container that is tolerant to sealed heating and it may then be heated in that state. In step b), heating may be performed at 100°C or higher, preferably at 105°C to 160°C, or more preferably 125°C to 145°C. In such a case, a sample may be sealed in a container that is tolerant to sealed heating and be heated. When heating is performed at 90°C to 100°C, a common polypropylene tube or the like can be adequately used as a container.

In order to elevate the temperature of a sample with higher certainty, the inside of the container may be conditioned to be free from a gas phase. For example, the container can be filled with a sample solution, so that air bubbles or the gas phase would not remain therein, and the container can then be hermetically sealed. Alternatively, a high-boiling-point solvent, such as mineral oil, may be introduced into the gas phase in the container.

When heating is performed at 90°C to 100°C in step b), a period of heat treatment needs to be approximately 5 minutes. If the temperature is elevated to 100°C or higher, however, a period of heat treatment may be less than 120 seconds. Preferably, a period of heat treatment may be less than 45 seconds, less than 35 seconds, or less than 25 seconds. Most preferably, a period of heat treatment may be 15 seconds or less. Heating may be performed by preheating the heating means to a designated temperature in advance and mounting the container on the heating means for a designated period of time only. After the completion of heating for a designated period of time, the sample may be cooled immediately.

The heated sample may be cooled to room temperature (e.g., 20°C to 30°C), refrigeration temperature (e.g., 2°C to 8°C), or ice cooling temperature. When it is difficult to perform nucleic acid amplification immediately after heating, in particular, the sample may be stored at refrigeration temperature or lower immediately after heating.

The step of heating may be performed at least two times. When a nucleic acid derived from a virus from the water environment is targeted, in contrast, the step of heating may be performed only once.

After steps a) and b), a sample that has been subjected to pretreatment for nucleic acid detection can be obtained.

### 4-4. Step c) Step of mixing with composition for nucleic acid detection

Step c) of the method of the present embodiment comprises mixing the sample after step b) with a composition for nucleic acid detection. When a target of nucleic acid detection is RNA, a composition for a reverse transcription polymerase chain reaction (RT-PCR) may be used as a composition for nucleic acid detection. When a target is DNA, a composition for a polymerase chain reaction (PCR) may be used as a composition for nucleic acid detection.

When a target of nucleic acid detection is RNA and a composition for RT-PCT is used as a composition for nucleic acid detection, the composition for RT-PCR can comprise, for example, the ingredients indicated below.
- DNA polymerase capable of reverse transcription (e.g., Tth DNA polymerase);
- at least a pair of primers for amplifying a region of interest of a template nucleic acid;
- deoxyribonucleoside triphosphate;
- divalent metal ion; and
- pH buffer.

When a target of nucleic acid detection is DNA and a composition for PCR is used as a composition for nucleic acid detection, the composition for PCR can comprise the ingredients indicated below.
- DNA polymerase (e.g., Taq DNA polymerase);
- at least a pair of primers for amplifying a region of interest of a template nucleic acid;
- deoxyribonucleoside triphosphate;
- divalent metal ion; and
- pH buffer.

When performing RT-PCR or PCR under real-time PCR conditions, the composition can further comprise a detection probe (e.g., TaqMan^{®} probe) or a fluorescent intercalator (e.g., SYBR^{®} GREEN). The composition may comprise internal reference DNA, uracil DNA glycosylase (UNG), or the like in order to perform detection/quantification with high accuracy.

The primers and the probes can be adequately designed in accordance with the structures of nucleic acids to be detected. The composition for RT-PCR or the composition for PCR may be prepared with the use of a commercially available kit or the like. Examples of known kits include PrimeScript^{(™)} RT Master Mix (Takara Bio Inc.), SuperScript III One-Step RT-PCR System with Platinum Taq DNA Polymerase (Thermo Fisher), and ReverTra Ace qPCR RT Master Mix (Toyobo Co., Ltd.).

After step b), the sample may be mixed with a composition for nucleic acid detection and subjected to the step of detection as early as possible, in order to avoid the influence of remaining nuclease activity or the like.

After step c), the concentration of EDTA in the composition for pretreatment and that in the detection composition may be designated in advance, so as to adjust the final concentration of EDTA to 0.001 to 10 mM in the sample subjected to the step of nucleic acid detection. In some embodiments, the final concentration of EDTA may be adjusted to 0.01 to 5 mM. In some other embodiments, it may be adjusted to 0.1 to 1 mM. The final concentration of EDTA can be, for example, 0.02 mM or higher, 0.03 mM or higher, 0.04 mM or higher, 0.05 mM or higher, 0.06 mM or higher, 0.07 mM or higher, 0.08 mM or higher, 0.09 mM or higher, 0.10 mM or higher, 0.11 mM or higher, 0.12 mM or higher, 0.13 mM or higher, 0.14 mM or higher, 0.15 mM or higher, 0.16 mM or higher, 0.17 mM or higher, 0.18 mM or higher, 0.19 mM or higher, 0.20 mM or higher, 0.21 mM or higher, 0.22 mM or higher, 0.23 mM or higher, 0.24 mM or higher, 0.25 mM or higher, 0.30 mM or higher, 0.35 mM or higher, 0.40 mM or higher, 0.45 mM or higher, 0.50 mM or higher, 0.60 mM or higher, 0.70 mM or higher, 0.80 mM or higher, or 0.90 mM or higher. The final concentration of EDTA can be, for example, 4.9 mM or lower, 4.8 mM or lower, 4.7 mM or lower, 4.6 mM or lower, 4.5 mM or lower, 4.4 mM or lower, 4.3 mM or lower, 4.2 mM or lower, 4.1 mM or lower, 4.0 mM or lower, 3.9 mM or lower, 3.8 mM or lower, 3.7 mM or lower, 3.6 mM or lower, 3.5 mM or lower, 3.4 mM or lower, 3.3 mM or lower, 3.2 mM or lower, 3.1 mM or lower, 3.0 mM or lower, 2.9 mM or lower, 2.8 mM or lower, 2.7 mM or lower, 2.6 mM or lower, 2.5 mM or lower, 2.4 mM or lower, 2.3 mM or lower, 2.2 mM or lower, 2.1 mM or lower, 2.0 mM or lower, 1.9 mM or lower, 1.8 mM or lower, 1.7 mM or lower, 1.6 mM or lower, 1.5 mM or lower, 1.4 mM or lower, 1.3 mM or lower, 1.2 mM or lower, 1.1 mM or lower, 1.0 mM or lower, 0.9 mM or lower, 0.8 mM or lower, 0.7 mM or lower, 0.6 mM or lower, or 0.5 mM or lower. By adjusting the concentration to the level indicated above, it is possible to prevent EDTA from inhibiting the reaction in that state. Alternatively, divalent metal salt may be present therein, so as to prevent EDTA from inhibiting the reaction.

When the final concentration of EDTA is 1 mM or higher, EDTA disadvantageously forms a chelate complex with an equivalent amount of divalent metal ions in the composition for RT-PCR or the composition for PCR. This may lower DNA polymerase activity. In such a case, 0.5 to 2.0 equivalent amounts of divalent metal salt relative to the amount of EDTA may be added before step d). Preferably, 0.75 to 1.25 equivalent amounts of divalent metal salt may be added.

### 4-5. Step d) Step of nucleic acid detection

Step d) of the present embodiment comprises detecting a nucleic acid in the sample after step c). A technique of nucleic acid detection is not particularly limited. Preferably, a method of nucleic acid amplification may be employed. When a target of nucleic acid detection is RNA, a method of RT-PCR may be employed as a method of nucleic acid amplification. When a target of nucleic acid detection is DNA, a method of PCR may be employed. Preferably, a method of real-time RT-PCR or a method of real-time PCR may be employed. Compositions that can be used for real-time RT-PCR and real-time PCR are as described in the "4-4. Step c) Step of mixing with composition for nucleic acid detection" section above.

When a method of real-time RT-PCR is performed, a step of detection is divided into a step of reverse transcription and a step of amplification and detection. The step of reverse transcription comprises generating complementary DNA (cDNA) from template RNA, and the step of amplification and detection comprises detecting a signal generated as a result of cDNA amplification. In the reaction and signal detection in the steps described above, for example, the CronoSTAR^{®} 96 Real-Time PCR System (Takara Bio Inc.) can be used.

Preferably, step d) may be initiated as early as possible after step b). A duration time from after step b) to step d) may be within 4 hours. Preferably, such duration time may be within 30 minutes.

According to the method of the present embodiment, a target nucleic acid in a sample can be detected in a convenient manner with high accuracy.

### Examples

### [Comparative Example 1] Detection of Pepper mild mottle virus (PMMoV) in treated sewage water

Sewage water (treated water) filtered through MF (microfiltration membrane) was introduced into a polypropylene centrifuge tube, and 20 µl thereof was mixed with 20 µl of a lysis buffer comprising 10 mM Tris-HCl (pH 8.0).

The centrifuge tube was placed in a heat block maintained at 140°C and heated for 15 seconds, 25 seconds, 35 seconds, or 45 seconds. A control sample that was not subjected to heat treatment was also prepared. The heated tube was cooled to room temperature and centrifuged at 10000× g for 1 minute. The supernatant was allowed to stand at room temperature for 3 minutes, 30 minutes, 4 hours, or 24 hours and then diluted to 10-fold with DNase/RNase-free water. A 10-µl fraction was separated, 10 µl of the cDNA Reverse Transcription Kit (Model: 4368813; ThermoFisher) was added and mixed therewith, and reverse transcription was carried out at 37°C for 60 minutes.

TaqPath^{®} qPCR (No. A15297; ThermoFisher) and the primers and the probe indicated below were added to 5 µl of the reverse transcription product to prepare a 25-µl reaction system, quantitative PCR (TaqManPCR) was performed using the Thermal Cycler Dice Real Time System (Takara Bio Inc.), and Ct was determined as the output value. PCR temperature conditions are as shown in Table 1.
Forward primer: GAGTGGTTTGACCTTAACGTTTGA (SEQ ID NO: 1)
Reverse primer: TTGTCGGTTGCAATGCAAGT (SEQ ID NO: 2)
Probe: FAM-CCTACCGAAGCAAATG-MGB-NFQ (SEQ ID NO: 3)

**Table 1**

| | Temperature (°C) | Time (sec.) |
|---|---|---|
| Denaturation | 95 | 20 |
| Annealing | 53 | 20 |
| Elongation | 72 | 20 |
| Number of cycles | 50 | |

Reverse transcription and quantitative PCR were carried out with a reference solution comprising a known amount of PMMoV under the same conditions, and Ct of the reference was compared with Ct of the sample to determine the quantified viral load per 1 µl and 5 µl.

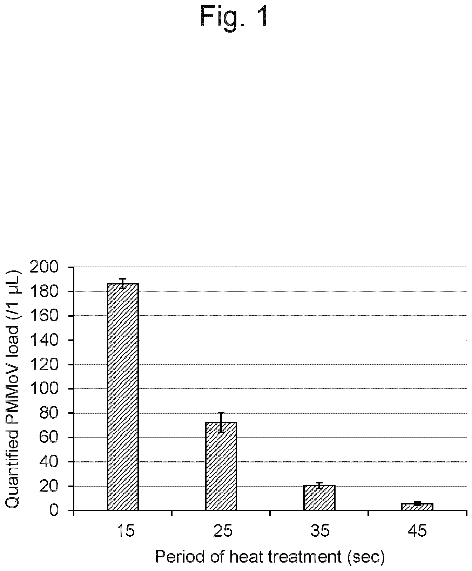

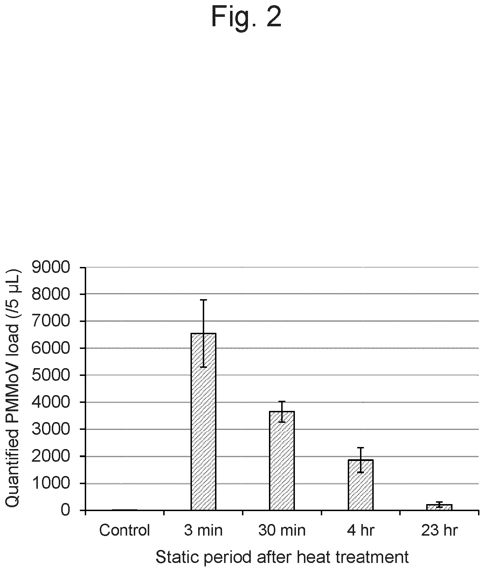

Fig. 1 shows the quantified viral loads (/1 µl) in the sample after relevant periods of heat treatment. The quantified viral load was found to become lower as the period of heat treatment became longer than 15 seconds. Fig. 2 shows a correlation between the static period from after 15-second heat treatment to before reverse transcription and the quantified PMMoV load (/5µl). The quantified viral load of the control sample that was not subjected to heat treatment is also shown. PMMoV was not detected in the sample that was not subjected to heat treatment. Heat treatment enables detection of PMMoV. As the duration time after heat treatment became longer, however, the quantified PMMoV load was found to be lower. Such a result is considered to occur due to the influence of a nuclease comprised in sewage water.

### [Comparative Example 2] Detection of purified PMMoV

*Nicotiana benthamiana* was inoculated with a viral solution comprising PMMoV, and leaves with mosaic symptoms were collected. The collected leaves were frozen at -80°C, and a phosphate buffer (pH 7.2) was added in an amount 10 times the weight of the virus-infected leaves. The leaves were completely fractured. Thereafter, the resultant was filtered through a 5-µm filter and a 0.45-µm filter, and a filtrate was obtained in a 1.5-ml Eppendorf^{®} tube. The filtrate was diluted to 10-fold with a lysis buffer comprising 10 mM Tris-HCl (pH 8.0).

The centrifuge tube was placed in a heat block maintained at 140°C and heated for 15 seconds, 25 seconds, 35 seconds, or 45 seconds. The heated tube was cooled to room temperature and centrifuged at 10000× g for 1 minute. The supernatant was immediately diluted to 10-fold with DNase/RNase-free water. A 10-µl fraction was separated, 10 µl of the cDNA Reverse Transcription Kit (Model: 4368813; ThermoFisher) was added and mixed therewith, and reverse transcription was carried out at 37°C for 60 minutes. The reverse transcription product (5 µl) was subjected to PCR under the same conditions as in Comparative Example 1.

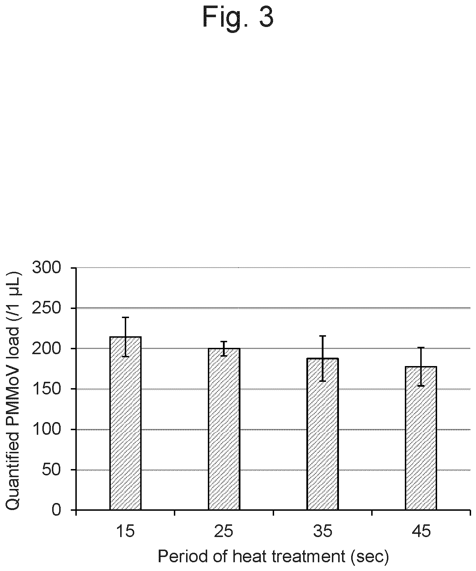

Fig. 3 shows the quantified viral loads (/1 µl) in the samples determined after relevant periods of heat treatment. In the case of a purified virus sample, no significant changes were observed in the quantified viral loads due to different periods of heat treatment. Such a result is considered to occur because, unlike sewage water, a purified virus sample is less likely to be influenced by a nuclease.

### [Example 1] Detection of PMMoV in treated sewage water in the presence of EDTA

Sewage water filtered through MF was introduced into a polypropylene centrifuge tube, and 20 µl thereof was mixed with 20 µl of a lysis buffer comprising 10 mM Tris-HCl (pH 8.0) and 5 mM EDTA.

The centrifuge tube was placed in a heat block maintained at 140°C and heated for 15 seconds. A control sample that was not subjected to heat treatment was also prepared. The heated tube was cooled to room temperature and centrifuged at 10000× g for 1 minute. The supernatant was allowed to stand at room temperature or 4°C for 3 minutes, 30 minutes, 4 hours, or 24 hours and then diluted to 10-fold with DNase/RNase-free water. A 10-µl fraction was separated, 10 µl of the cDNA Reverse Transcription Kit (Model: 4368813; ThermoFisher) was added and mixed therewith, and reverse transcription was carried out at 37°C for 60 minutes. The reverse transcription product (5 µl) was subjected to PCR under the same conditions as in Comparative Example 1.

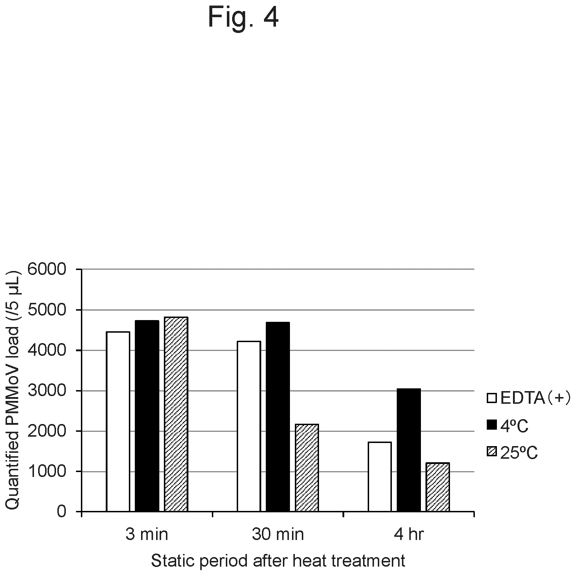

Fig. 4 shows a correlation between the period from finish of heat treatment to start of reverse transcription and the quantified PMMoV load (/5 µl) of the sample that was allowed to stand at room temperature after heat treatment with the addition of EDTA, that of the sample that was allowed to stand at room temperature after heat treatment without the addition of EDTA, and that of the sample that was allowed to stand at 4°C after heat treatment without the addition of EDTA. Without the addition of EDTA, the sample that was allowed to stand at room temperature was found to exhibit a significant lowering in the quantified PMMoV load 30 minutes later and 4 hours later, compared with the sample that was allowed to stand at 4°C. With the addition of EDTA, in contrast, no significant differences were observed in quantified viral loads between the sample that was allowed to stand at room temperature and the sample that was allowed to stand at 4°C 30 minutes later. In addition, the quantified viral loads with EDTA were higher than the quantified viral loads without EDTA 4 hours later. This indicates that the addition of EDTA improves stability of the sample.

### [Example 2] Comparison of results of detection of PMMoV in treated sewage water depending on heating conditions

The 3 types of sewage water samples (Samples A to C) after filtration were each suspended in 20 µl of a lysis buffer comprising 10 mM Tris-HCl (pH 8.0) and 5 mM EDTA in polypropylene centrifuge tubes.

The centrifuge tube was placed in a heat block maintained at 140°C and heated for 15 seconds, or the centrifuge tube was placed in a heat block maintained at 95°C and heated for 5 minutes. The heated tubes were cooled to room temperature and centrifuged at 10000× g for 1 minute. The supernatants were allowed to stand at room temperature for 30 minutes, 10 µl of the cDNA Reverse Transcription Kit (Model: 4368813; ThermoFisher) was added and mixed therewith, and reverse transcription was carried out at 37°C for 60 minutes. The reverse transcription product (5 µl) was subjected to PCR under the same conditions as in Comparative Example 1.

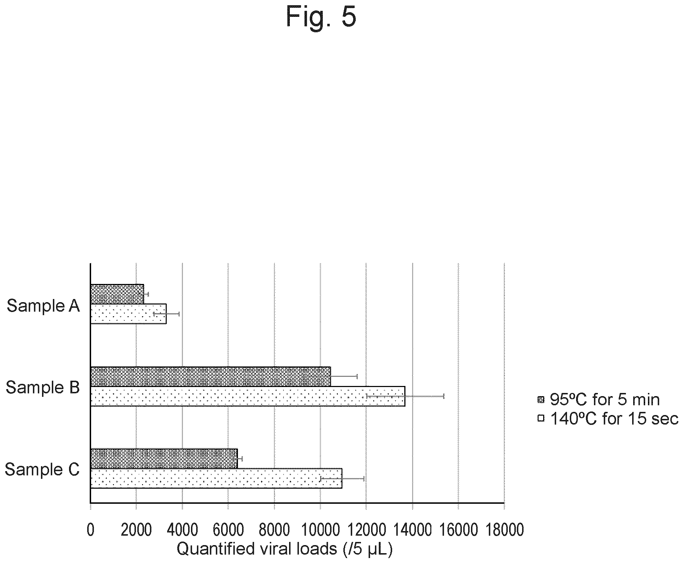

Fig. 5 shows the quantified PMMoV loads (/5 µl) of Samples A to C under each of the heating conditions. High quantified viral loads were obtained as a result of treatment at 140°C for 15 seconds. This indicates that a sample can be treated within a short period of time by elevating the temperature to 140°C.

### [Example 3] PCR at various EDTA concentrations and divalent metal ion concentrations

Genomic DNA extracted from *Bacillus subtilis* (No. 3134, obtained from NBRC) with the use of the QIAamp DNA Kit (56304, QIAGEN) was dissolved to 100 pg/µl in DNase/RNase-free water to obtain a DNA solution. The genomic DNA was fractionated to 200 pg/tube with the use of TB Green premix Ex TaqII (No. RR820S, Takara Bio Inc.), and EDTA and FeSO₄ were added thereto to the concentrations shown in Table 3. The primers indicated below were added to prepare a 20-µl reaction system, and quantitative PCR was carried out with the use of the Thermal Cycler Dice Real Time System (Takara Bio Inc.). PCR temperature conditions are as shown in Table 2.
Forward primer: TCAACTAGTTCAGTATGGACGAC (SEQ ID NO: 4)
Reverse primer: CCTCATCAAGAAACCACTGA (SEQ ID NO: 5)

**Table 2**

| | Temperature (°C) | Time (sec.) |
|---|---|---|
| Hot start | 95 | 120 |
| Denaturation | 95 | 30 |
| Annealing/Elongation | 60 | 30 |
| Number of cycles | 45 | |

Table 3 shows the Ct numbers under various conditions. When the final concentration of EDTA was 0 to 0.33 mM, it was possible to perform nucleic acid amplification without the addition of FeSO₄. When the concentration of FeSO₄ exceeded 1.0 mM, in contrast, nucleic acid amplification was not observed. When the final concentration of EDTA was 1 mM, nucleic acid amplification was observed at a smaller Ct number with the addition of FeSO₄ at 0.33 to 1 mM. When the final concentration of EDTA was 3.3 mM, it was possible to perform nucleic acid amplification with the addition of FeSO₄ at 1.0 to 3.3 mM. When the final concentration of EDTA was 10 mM, nucleic acid amplification was not observed with the addition of FeSO₄.

**Table 3**

| Sample No. | PCR final concentration (mM) | | Ct number |
|---|---|---|---|
| | EDTA | FeSO₄ | |
| 1 | 0 | 0 | 22.4 |
| 2 | 0.20 | 0 | 22.4 |
| 3 | 0.33 | 0 | 22.3 |
| 4 | 1.0 | 0 | 25.8 |
| 5 | 3.3 | 0 | - |
| 6 | 10 | 0 | - |
| 7 | 0 | 0.33 | 23 |
| 8 | 0.20 | 0.33 | 22.4 |
| 9 | 0.33 | 0.33 | 22.3 |
| 10 | 1.0 | 0.33 | 22.5 |
| 11 | 3.3 | 0.33 | - |
| 12 | 10 | 0.33 | - |
| 13 | 0 | 1.0 | - |
| 14 | 0.20 | 1.0 | - |
| 15 | 0.33 | 1.0 | - |
| 16 | 1.0 | 1.0 | 22.5 |
| 17 | 3.3 | 1.0 | - |
| 18 | 10 | 1.0 | - |
| 19 | 0 | 3.3 | - |
| 20 | 0.20 | 3.3 | - |
| 21 | 0.33 | 3.3 | - |
| 22 | 1.0 | 3.3 | - |
| 23 | 3.3 | 3.3 | 22.8 |
| 24 | 10 | 3.3 | - |

As described above, EDTA would not influence nucleic acid amplification at low concentration. In a reaction system comprising EDTA at high concentration, in contrast, nucleic acid amplification would not be sufficiently performed. On the other hand, the addition of divalent metal ions at adequate concentration at a timing after heating and before nucleic acid amplification was found to recover the adverse influence of EDTA.

## Claims

1. A method for pretreatment of a target sample of nucleic acid detection comprising:
i) a step of mixing a sample with a composition for pretreatment comprising ethylenediaminetetraacetic acid (EDTA); and
ii) a step of heating the sample mixed in step i) using a heating means maintained at 90°C or higher.

2. The method according to claim 1, wherein the sample is a water sample in the environment.

3. The method according to claim 1, wherein the nucleic acid is a viral nucleic acid.

4. The method according to claim 1, which comprises a step of concentrating nucleic acids in a sample before step i).

5. The method according to claim 1, wherein the concentration of EDTA in the sample mixed in step i) is 0.01 to 20 mM.

6. The method according to claim 5, wherein the concentration of EDTA in the sample mixed in step i) is 0.1 to 10 mM.

7. The method according to claim 1, wherein a duration time of heat treatment in step ii) is less than 120 seconds.

8. The method according to claim 1, wherein temperature of the heating means used in step ii) is 105°C to 160°C.

9. A method for detecting a nucleic acid in a sample comprising:
a) a step of mixing a target sample of nucleic acid detection with a composition for pretreatment comprising EDTA;
b) a step of heating the sample after step a) using a heating means maintained at 90°C or higher;
c) a step of mixing the sample after step b) with a composition for nucleic acid detection; and
d) a step of detecting a nucleic acid in the sample after step c).

10. The method according to claim 9, wherein the composition for nucleic acid detection is a composition for a reverse transcription polymerase chain reaction (RT-PCR).

11. The method according to claim 9, wherein a duration time from after step b) to before step d) is less than 4 hours.

12. The method according to claim 9, wherein the concentration of EDTA in the sample mixed in step c) is 0.001 to 10 mM.

13. A composition comprising EDTA at 0.1 to 10 mM and a pH buffer, which is used for pretreatment of a water sample in the environment serving as a target of viral nucleic acid detection.
